# EUROPEAN PATENT APPLICATION

(11) **EP 3 378 421 A1**
(43) Date of publication of application: **26.09.2018**
(21) Application number: 17162730.0
(22) Date of filing: 24.03.2017
(51) Int. Cl.: A61B 17/34, A61M 39/02, A61B 5/02, A61B 90/98, A61B 5/00

(54) **ENDOVASCULAR DEVICE NAVIGATION**

(71) Applicant: Koninklijke Philips N.V., 5656 AE Eindhoven (NL)
(72) Inventor: KAHLERT, Joachim, 5656 AE Eindhoven (NL); MUELLER, Manfred, 5656 AE Eindhoven (NL)
(74) Representative: de Haan, Poul Erik

(57) **Abstract**

The present invention discloses an endovascular device arrangement (1) comprising a vascular access port (10); an endovascular device (20) for inserting into a patient's blood vessel through said vascular access port; and a near-field communication module (15) including an antenna (17), wherein at least the antenna of the near-field communication module is located within the vascular access port; and the endovascular device comprises an electronic device (25) and an antenna arrangement (21) communicatively coupled to said electronic device for maintaining a near-field communication link between the electronic device and the near-field communication module whilst the endovascular device passes through the vascular access port. Also disclosed are an endovascular device (20) and a vascular access port (10) for use in such an endovascular device arrangement (1), an endovascular intervention system including such an endovascular device arrangement (1) and a method of operating such an endovascular intervention system.

## Description

### FIELD OF THE INVENTION

The present invention relates to an endovascular device arrangement comprising a vascular access port and an endovascular device for inserting into a patient's blood vessel through said vascular access port, as well as to an endovascular device and a vascular access port respectively for use in such an endovascular device arrangement.

The present invention further relates to an endovascular intervention system including such an endovascular device arrangement.

The present invention still further relates to a method of operating such an endovascular intervention system.

### BACKGROUND OF THE INVENTION

Data acquired by a sensor integrated in an endovascular device such as a pressure wire typically requires a wired interconnection to power the device and to transfer the data from the device to a user console, in order to record, post process, monitor and/or visualize the data. Medical professionals such as cardiologists are trained to navigate guide wires and catheters through the human body, e.g. through the heart, by gently advancing and rotating (torqueing) the endovascular device through the patient's vascular system.

The handling and feedback properties from the endovascular device are essential for fast and safe navigation. Any cables or other attachments to the endovascular device hinder such navigation. For this reason, endovascular devices typically can be untethered from such attachments. This means that the endovascular device itself can be disconnected from and reconnected to the wires that connect it to the external user console.

Still, even when comprising such tethering functionality, the handling capabilities of endovascular devices that include sensors are typically compromised. The tethering functionality typically requires bulky connectors, whilst disconnecting and reconnecting the endovascular device from and to its connecting wires takes time and often influences signal quality. This lengthens procedures and increases the risk to the patient.

Hence, wired connections to an interventional medical device such as an endovascular device presents an ergonomic and workflow burden for the physicians using the device. Furthermore, wired connectors and galvanic connections to such an interventional tool present a hygiene concern, e.g. when interconnecting sterile and non-sterile components. US 2016/0183793 A1 discloses a Wireless Catheter Module (WCM) adapted for attachment to a diagnostic catheter to enable the bio-electrical signals, analog signals picked up by the probing electrodes imbedded in the attached catheter, to be captured and digitized by A-to-D converter electronics in the WCM whereupon packets of the digitized signals can then be transmitted wirelessly from the WCM to a remote Wireless Base Station (WBS) forming a receiver component of the present system. The WBS is adapted for operative connection to an EP recording and cardiac stimulation system.

Although the WCM allows use of the diagnostic catheter without it being connected to a cable or the like, the positioning of the relatively bulky WCM at its proximal end still hampers the navigation of the diagnostic catheter.

### SUMMARY OF THE INVENTION

The present invention seeks to provide an endovascular device arrangement that allows its operator to freely manoeuver the endovascular device inside a patient, as well as to provide an endovascular device and a vascular access port respectively for use in such an endovascular device arrangement.

The present invention further seeks to provide an endovascular intervention system including such an endovascular device arrangement.

The present invention yet further seeks to provide a method of operating such an endovascular intervention system.

According to an aspect, there is provided an endovascular device for inserting into a patient's blood vessel through a vascular access port comprising a near-field communication module including an antenna, wherein at least the antenna of the near-field communication module is located within the vascular access port, the endovascular device comprising an electronic device and an antenna arrangement communicatively coupled to said electronic device for maintaining a near-field communication link between the electronic device and the near-field communication module whilst the endovascular device passes through the vascular access port.

The present invention is based on the insight that an endovascular device can be freely operated by a clinician without the requirement of it being connected to a user console through a cable or wireless communication module by providing the vascular access port through which the endovascular device is entered into the patient with NFC capability whilst providing the endovascular device with an antenna arrangement that allows for the NFC link between the vascular access port and the endovascular device to be maintained as the endovascular device moves through the vascular access port, thereby overcoming the intrinsic distance limitations associated with NFC protocols.

For example, the endovascular device may comprise an antenna arrangement including a plurality of antenna elements distributed along the elongation direction of the endovascular device, such as along a shaft of the endovascular device such that regardless of the total length of the endovascular device that is inserted within the patient, there is always an antenna element located in close proximity to the antenna of the NFC module in the vascular access port such that the NFC link between the NFC module and the endovascular device can be maintained. To this end, the antenna arrangement may comprise an array of antenna elements, each of said antenna elements being communicatively coupled to said electronic device.

In a preferred embodiment, the endovascular device further comprises a detection element configurably coupled in between the electronic device and the antenna elements, said detection element being adapted to detect the antenna element of said array proximal to the vascular access port and to selectively connect said detected antenna element to the electronic device. This ensures that only one of the antenna elements of the array is communicatively coupled to the electronic device at any given point in time, thereby avoiding the risk of interference between a primary induced signal by an antenna element proximal to the vascular access port, i.e. closest to the antenna of the NFC module, and secondary induced signals by antenna elements further away but still in range of this antenna.

The detection element may be adapted to periodically or continually check which of said antenna elements is proximal to the vascular access port such that the selected antenna element proximal to the vascular access port can be regularly updated, thereby reducing the risk that the NFC link between the NFC module and the electronic device of the endovascular device is (temporarily) broken when the endovascular device is moved through the vascular access port causing a previously selected antenna element to move out of range of the antenna of the NFC module.

In an embodiment, the detection element may be adapted to detect the antenna element of said array proximal to the vascular access port based on the strength of an induced signal received by said detected antenna element, which is a particularly straightforward manner of detecting which of the antenna elements of the array of antenna elements on the endovascular device is closest to the antenna of the NFC module associated with the vascular access port through which the endovascular device is moved into the patient.

In a particular embodiment, the electronic device comprises a sensor. In this embodiment, the endovascular device, e.g. the electronic device, may be adapted to communicate the sensor data to the NFC module in a unidirectional fashion, e.g. as a modulation on the current induced by the antenna arrangement of the endovascular device from the electromagnetic field generated by the NFC module.

In another particular embodiment, the electronic device comprises an actuator for interacting with the patient's tissue, for example to transfer electrical energy into mechanical motion, which actuator may be used to control an interventional tool at the distal tip of the endovascular device such as a biopsy needle, or to deliver a stimulus to the tissue. In this embodiment, no data may need to be transferred from the endovascular device to the NFC module. Instead, the NFC module may be responsive to a user command provided through a user console communicatively coupled to the NFC module in order to trigger the actuation of the actuator once the endovascular device is correctly positioned within the patient.

According to another aspect, there is provided a vascular access port for communicating with such an endovascular device, the vascular access port comprising a near-field communication module including an antenna, wherein at least the antenna of the near-field communication module is located within the vascular access port, in order to facilitate wireless operation of the endovascular device as previously explained.

The near-field communication module may be arranged to generate a plurality of frequency bands in a frequency multiplex schema, in order to minimize the risk of interference between the NFC communication link and other signals within the space in which the endovascular device arrangement is being used.

The near-field communication module may be connected to a user console in any suitable manner, e.g. using a wireless connection. Alternatively, the vascular access port may further comprise a connection cable for connecting the near-field communication module to a user console for the endovascular device.

According to a further aspect, there is provided an endovascular device arrangement including such an endovascular device and such a vascular access port, thereby providing a complete solution facilitating wireless operation, e.g. insertion, of an endovascular device into a patient.

According to another aspect, there is provided an endovascular intervention system including the endovascular device arrangement of any of the herein described embodiments and a user console adapted to communicate with the electronic device through the near-field communication module. Such an endovascular intervention system may be operated by a clinician without requiring a wired or direct wireless connection between the endovascular device and the user console, e.g. using a wireless communication module that needs connecting to the endovascular device, thereby improving the ease by which the endovascular device can be manipulated, e.g. within the patient as the endovascular device is moved through the vascular access port.

According to yet another aspect, there is provided a method of operating such an endovascular intervention system, the method comprising maintaining a near-field communication link between the near-field communication module and the electronic device during passing of the endovascular device through the vascular access port. Consequently, communication between the endovascular device and the user console can be maintained without the requirement of a wired or dedicated wireless connection between the endovascular device and the user console, thereby improving the ease of use of the endovascular device within the patient as previously explained. The near field communication link in some embodiments may be a unidirectional data communication from the electronic device to the near-field communication module; and/or an energy transfer from the near-field communication module to the electronic device.

In a preferred embodiment, the method further comprises identifying the antenna element of the array of antenna elements proximal to the vascular access port such that the NFC link between the NFC module and the endovascular device can be automatically maintained as the endovascular device is moved through the vascular access port. This preferably includes selectively coupling said identified antenna element to the electronic device in order to avoid interference between the induced current generated with the identified antenna element and other antenna elements within range of the antenna of the NFC module.

### BRIEF DESCRIPTION OF THE DRAWINGS

Embodiments of the invention are described in more detail and by way of nonlimiting examples with reference to the accompanying drawings, wherein:
FIG. 1 schematically depicts an endovascular device arrangement according to an example embodiment;
FIG. 2 schematically depicts an endovascular device arrangement according to an example embodiment in circuit diagram form;
FIG. 3 schematically depicts an example embodiment of an endovascular device;
FIG. 4 schematically depicts another example embodiment of an endovascular device; and
FIG. 5 schematically depicts an example embodiment of an endovascular intervention system including the endovascular device arrangement according to embodiments of the present invention.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

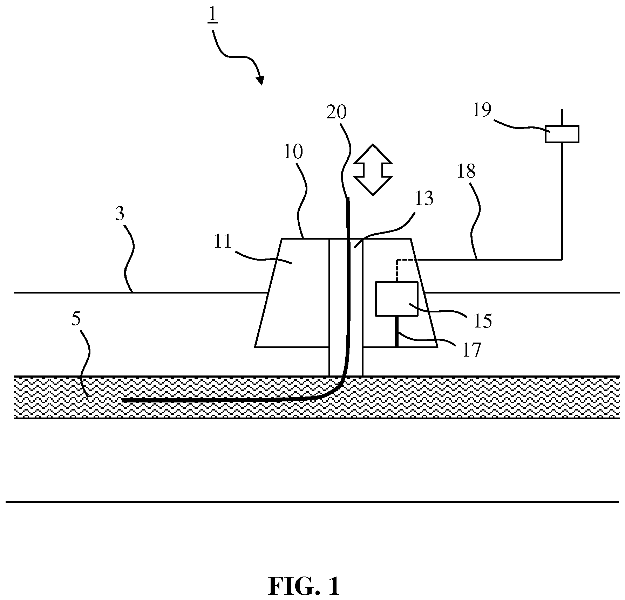

FIG. 1 schematically depicts an endovascular device arrangement 1 according to an example embodiment. The endovascular device arrangement 1 comprises a vascular access port 10, which may be fitted by a clinician into a body portion 3 of a patient to provide access to a blood vessel 5, e.g. an artery, vein, or the like, of the patient as is well-known per se. For example, the vascular access port 10 may provide access to the blood vessel 5 for an endovascular device 20, such as a catheter or a guide wire, which may be inserted into the blood vessel 5 by a clinician through a channel 13 in the vascular access port 10, upon which the clinician may manipulate the endovascular device 20, e.g. push in, pull-out and/or twist (torque) as indicated by the block arrow, in order to move the endovascular device 20 into a desired location within the patient's body, e.g. the patient's heart. This for example may be done by the clinician in order to manoeuver a distal tip of the endovascular device 20 into a desired location, for example in case the distal tip comprises an electronic device such as a sensor to obtain sensor readings of interest of the desired location or an actuator to actuate an interventional tool, e.g. to obtain a biopsy sample or to perform an interventional procedure.

In order to ensure that the clinician can manoeuver the endovascular device 20 within the patient's body as freely as possible, the endovascular device arrangement 1 is arranged to implement a near-field communication (NFC) protocol between the vascular access port 10 and the endovascular device 20 such that the endovascular device 20 does not need to be connected to a wireless communication module or a cable in order to facilitate communication between the endovascular device 20 and a user console connected thereto. To this end, the housing 11 of the vascular access port 10 through which the channel 13 extends contains a NFC module 15 coupled to an antenna 17 for communicating with an antenna arrangement on the endovascular device 20 as will be explained in further detail below. The NFC module 15 may be coupled to a user console, e.g. in a wireless fashion or through a wired connection 18, e.g. a cable 18, which wired connection 18 may comprise any suitable connection element 19, e.g. a plug or the like, for connecting the NFC module 15 to the user console of an endovascular intervention system including the endovascular device arrangement 1 as will be described in further detail below. The housing 11 of the vascular access port 10 may be made of any suitable (biocompatible) material, such as a polymer material, stainless steel, titanium, or the like. The NFC module 15 may be integrated inside the housing 11 in any suitable manner, as will be readily understood by the skilled person, such that this integration is not explained in further detail for the sake of brevity only.

Alternatively, only the antenna 17 of the NFC module 15 is located within the vascular access port 10, with the NFC module 15 itself being located outside the vascular access port 10, e.g. on an external surface of the vascular access port 10 as in order to be able to establish the NFC link between the NFC module 15 and the endovascular device 20, only the antenna 17 needs to be located within the vascular access port 10.

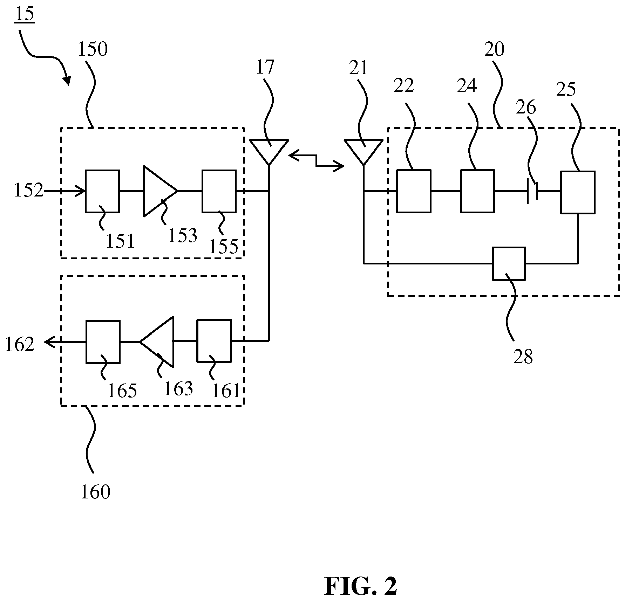

FIG. 2 schematically depicts a circuit diagram of the endovascular device arrangement 1 according to an example embodiment. The endovascular device arrangement 1 comprises the NFC module 15 in the vascular access port 10 and an endovascular device 20 for passing through the vascular access port 10 as explained above. The endovascular device 20 includes an electronic device 25 incorporated on the endovascular device 20 for supporting endovascular procedures or investigations. Such an electronic device for example may provide sensor capability to the endovascular device 20. In an embodiment, the sensor is a pressure sensor although it should be understood that different types of sensors are equally feasible. Moreover, the electronic device 25 is not limited to sensors. In an alternative embodiment, the electronic device 25 is an actuator, e.g. of an element to apply energy such as an electric current to tissue, e.g. to stimulate the tissue, or of an element to mechanically interact with tissue, e.g. a biopsy needle or the like. The electronic device 25 may be located in any suitable location on the endovascular device 20, such as at the distal tip of the endovascular device 20, e.g. in order to provide a 'look-ahead' sensing functionality in case of the electronic device 25 being a sensor.

The NFC module 15 in some embodiments comprises a transmission stage 150 and a reception stage 160, although in alternative embodiments the reception stage 160 may be omitted, e.g. where the NFC module 15 only supplies power to the electronic device 25, e.g. in case of the electronic device 25 being an actuator. The transmission stage 150 is driven by input power 152, which input power may be received from a user console coupled to the vascular access port 10, e.g. via one or more wires in the connection cable 18. The NFC module 15 may have any suitable design. For example, the NFC module 15 may include an oscillator 151, a power amplifier 153 and a matching circuit 155. The oscillator 151 may be configured to generate a desired frequency output, e.g. a frequency spectrum containing multiple frequency bands for a frequency multiplexing schema such as FDMA, for example to reduce interference on the NFC communication as is well-known per se. The oscillator signal may be amplified by the power amplifier 124 as is also well-known per se and will therefore not be further explained for the sake of brevity only. The matching circuit 155 may be included to filter out harmonics or other unwanted frequencies and match the impedance of the NFC module 15 to the antenna 17. It should be understood that the foregoing describes an example embodiment of such a transmission stage 150 and that alternative embodiments, e.g. comprising additional components, although not explicitly described, are equally feasible.

The reception stage 160 may be adapted to receive a modulated version of the frequency output generated with the transmission stage 150, which modulation may have been generated with a modulation stage 28 of the electronic device 25 of the endovascular device 20. Such a modulation for example may include sensor data acquired with the electronic device 25 being a sensor into the frequency output produced by the transmission stage 150 of the NFC module 15. Any suitable type of modulation protocol may be used for this purpose, such as on-off keying (OOK). In accordance with well-known NFC principles, such modulation may take an encoded form, e.g. pulse-interval encoding (PIE) or Manchester encoding in order to ensure that the power supply to the electronic device 25 is not disrupted in case the binary data value produced by the electronic device 25 does not change over a prolonged period of time. The reception stage 160 may comprise a matching circuit 161, an amplifier 163 and a demodulator 162 to extract the modulation from the frequency output produced by the transmission stage 150, which modulation may be provided as an output signal 162 to the user console connected to the vascular access port 10, e.g. through the connection cable 18, for processing and visualization of the data acquired with the electronic device 25 as represented by this modulation.

This scenario for example may be deployed if the endovascular device 20 is to be operated in a passive mode. Alternatively, the endovascular device 20 may be configured as an active NFC device, in which case a time-divisional multiplexing schema may be deployed in which during a first time period the endovascular device 20 is powered using the NFC module 15 and in a second time period following the first time period the endovascular device 20 generates a transmission to be received by the NFC module 15, which transmission typically comprises data collected with the electronic device 25, e.g. a sensor or the like. In such an active scenario, the endovascular device 20 may further comprise its own transmission stage (not shown), which may be powered by an energy storage device 26 such as a capacitor, a rechargeable battery, or the like, which energy storage device 26 harvests energy provided by the NFC module 15 during the first time period. The energy storage device 26 may be further coupled to the electronic device 25 to ensure that the electronic device 25 can continue is operation during the data transfer period, i.e. the second time period. In such an embodiment, the endovascular device 20 may further comprise a data storage element (not shown) into which data from the electronic device 25 is stored, which stored data is transferred during the second time period. This for example is advantageous if the electronic device collects data during the first time period (as well as during the second time period) such that the data collected during the first time period can be temporarily stored in the data storage device before transferring it to the NFC module 15. Alternatively, the data storage element may be omitted if the electronic device 25 is only required to generate data during the second time period, in which case this data can be immediately transferred over the NFC link with the NFC module 15.

As will be understood by the skilled person, the energy storage device 26 may also be included in the design of the endovascular device 20 where this device is to be operated as a passive NFC device, e.g. to smooth the power supply to the electronic device 25. The endovascular device 20 may further comprise any suitable circuit element, such as a matching circuit 22 and a rectifier 24, for processing the frequency signal generated with the NFC module 15. To this end, the endovascular device 20 comprises an antenna arrangement 21 coupled to the electronic device 25, e.g. via one or more further circuitry components as schematically depicted in FIG. 2, which antenna arrangement 21 is arranged such that the NFC communication between the NFC module 15 and the endovascular device 20 can be maintained as the endovascular device 20 is moved in or out the vascular access port 10.

A particular challenge addressed by embodiments of the present invention is that NFC technology is a short range communication technology (typically over distances of less than 10 cm) such that maintaining the NFC link between the NFC module 15 in the vascular access port 10 and the endovascular device 20 is not straightforward when the endovascular device 20 is displaced by the clinician, e.g. pushed in or pulled out of the patient's body, thereby altering the distance between the transmit antenna 17 of the NFC module 15 and any antenna element on the endovascular device 20. In particular, where these distance is increased, this may lead to the loss of this NFC link, which would lead to a disruption of the operation of the electronic device 25 and the transmission of data (if applicable) from the electronic device 25 to the NFC module 15.

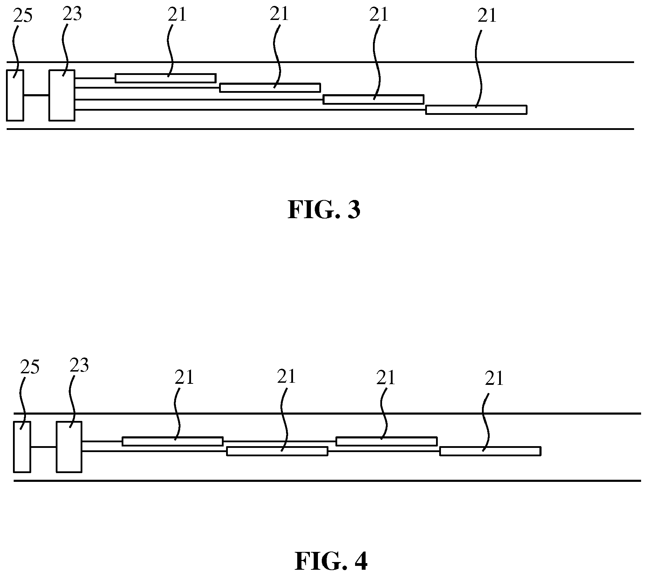

To this end, in at least some embodiments the endovascular device 20 comprises an array of antenna elements 21 as schematically depicted in FIG. 3, where the antenna elements 21 are distributed along an elongation direction of the endovascular device 20, such as along the shaft of the endovascular device 20. Consequently, such an arrangement of distributed antenna elements 21 ensures that there is always an antenna element 21 on the endovascular device 20 that is proximal to the vascular access port 10, i.e. is located at least partially within this vascular access port. The channel 13 of the vascular access port is dimensioned such that where such an antenna element 21 is at least partially located within the vascular access port 10, the distance between this antenna element and the NFC module 15 is small enough to reliably establish a NFC link between the NFC module 15 and the electronic device 25.

Any suitable type of antenna element 21 may be used for this purpose. For example, the antenna elements 21 may each be configured as a loop antenna comprising an air core or a physical core such as a ferrite core. Transfer of energy between the NFC module 15 and the endovascular device 20 may occur by coupling energy from the near-field of the transmitting antenna 17 to the receiving antenna element 21 at least partially residing within the vascular access port 10. Each antenna element 21 may have a resonance frequency based on the inductance and capacitance of the antenna element. For example, where the antenna element 21 is a loop antenna, the inductance typically is created by the loop, whereas one or more capacitors (not shown) may be added to the loop antenna's inductance to create a resonant structure at a desired resonant frequency.

The antenna elements 21 may be spaced with a minimal spacing in between each other to ensure that whatever the position of the endovascular device 20 relative to the vascular access port 10, there is always an antenna element 21 at least partially within the vascular access port 10 such that the NFC link between the NFC module 15 and the endovascular device 20 (i.e. the electronic device 25) can be maintained. This however may lead to a situation in which the neighboring antenna elements 21 of the antenna element 21 at least partially within the vascular access port 10 also sense the electromagnetic field generated by the NFC module 15 through its transmit antenna 17, which may lead to secondary inductions by these neighboring antenna elements 21 in addition to the primary induction generated by the antenna element 21 proximal to the vascular access port 10.

In order to avoid interference between such primary and secondary inductions, which for example may become problematic where the induced current is modulated with data generated with the electronic device 25 as explained above, the endovascular device 20 may further comprise a detection element 23 configurably coupled in between the electronic device 25 and the antenna elements 21, said detection element being adapted to detect the antenna element 21 of the array of antenna elements that is located proximal to, i.e. at least partially within, the vascular access port 10. For example, the detection element 23 may be adapted to detect the antenna element of said array proximal to the vascular access port based on the strength of an induced signal generated by the detected antenna element 21 from the electromagnetic field generated by the NFC module 15. To this end, the detection elements 23 may comprise one or more switches that can switch between a connected state and a disconnected state, such that each antenna element 21 can be selectively connected to the electronic device 25 (and any of the other previously described circuit elements of the endovascular device 20). The detection element 23 may be powered by the currents induced with the antenna elements 21 from the electromagnetic field generated by the NFC module 15 and may be adapted to periodically check which of the antenna elements 21 is located at least partially within the vascular access port 10, such that the detection element 23 can change the selected antenna element 21 upon translation of the endovascular device 20 through the vascular access port 10 as previously explained. Any suitable frequency for performing this period check may be used. For example, the frequency F may be based on a typical maximum translation speed V (in m/s) of the endovascular device 20 through the vascular access port 10 and a spacing S (in m) between the antenna elements 21, e.g. F = V*S.

Alternatively, the detection element 23 may be responsive to a motion sensor (not shown) such as an accelerometer or a gyroscope, which motion sensor maybe powered by the current induced by the antenna arrangement of the endovascular device 20 as previously explained, such that the detection element 23 only reinvestigates which of the antenna elements 21 is proximal to the vascular access port 10 upon the motion sensor detecting a displacement of the endovascular device 20, which may be indicative of the endovascular device 20 being pushed into the patient or pulled out of the patient the vascular access port 10.

In FIG. 3, each antenna element 21 is individually connected to the detection element 23. However, in an alternative embodiment schematically depicted in FIG. 4, the antenna elements 21 may be divided into groups, with each group of antenna elements 21 being serially connected to the detection element 23. In this embodiment, the spacing between neighboring antenna elements 21 in each group is such that when one of the antenna elements 21 of the group is located at least partially within the vascular access port 10, none of its neighboring antenna elements in the group are close enough to the NFC module 15 to generate a secondary induced current that is strong enough to interfere with the primary induced current by the antenna element 21 proximal to the vascular access port 10. The latter embodiment has the advantage that fewer interconnections between the antenna elements 21 and the detection module 23 extending along the endovascular device 20 are required, which may be beneficial in application domains in which the form factor of the endovascular device 20 is particularly critical.

At this point it is noted that although the vascular access port 10 and the endovascular device 20 are described as part of an endovascular device arrangement 1, it should be understood that the vascular access port 10 and the endovascular device 20 maybe provided as separate entities.

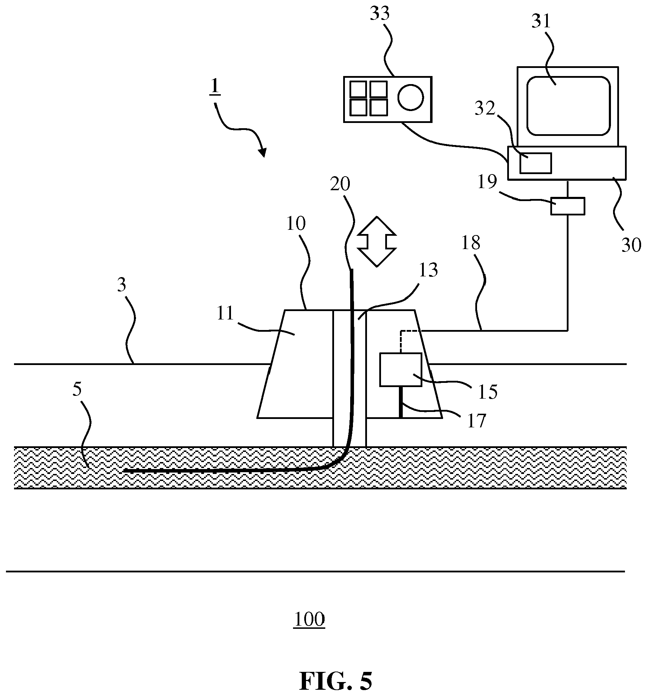

FIG. 5 schematically depicts an endovascular intervention system 100 including the above described endovascular device arrangement 1 and a user console 30 communicatively coupled to the NFC module 15, here through a connection cable 18 although it should be understood that other suitable communicative couplings, e.g. wireless communicative couplings between the NFC module 15 and the user console 30, equally may be contemplated. The user console 30 may comprise a processor arrangement 32 including one or more processors such as a signal processor, adapted to process signals received from the NFC module 15, e.g. sensor signals provided by the electronic device 25 being a sensor such as a pressure sensor. The user console 30 may further comprise or be connected to a display device 31 under control of the processor arrangement 32 for visualization of the processed signals received from the NFC module 15 as well as a user interface 33, which may be used by a user of the endovascular intervention system 100 to control the processor arrangement 32, e.g. to select a particular visualization mode of the processed signals received from the NFC module 15. The processor arrangement 32 may be further adapted to control the NFC module 15, e.g. to provide its transmission stage 150 and/or its reception stage 160 with control signals to control the operation of the respective elements of these respective stages, as explained in more detail above.

The endovascular intervention system 100 typically is operable in accordance with a method according to at least one embodiment of the present invention, in which the NFC link between the NFC module 15 and the endovascular device 20 is maintained as the endovascular device 20 is translated (pushed in or pulled out) through the vascular access port 10 as explained in more detail above. The NFC link in preferred embodiments is a unidirectional link in which power is transferred from the NFC module 15 to the endovascular device 20 and, where a data transfer is required, unidirectional data transfer from the endovascular device 20 to the NFC module 15 as explained in more detail above. It is reiterated for the avoidance of doubt that such unidirectional data transfer may be achieved with the endovascular device 20 being a passive NFC device or an active NFC device as previously explained.

In order to maintain the NFC link between the NFC module 15 and the endovascular device 20, the method may further comprise identifying the antenna element 21 of the array of antenna elements of the endovascular device 20 that is located proximal to the vascular access port 10, i.e. at least partially within the vascular access port 10, and selectively coupling the identified antenna element 21 to the electronic device 25, e.g. using a switch arrangement between the antenna elements 21 and the electronic device 25, which switch arrangement may be located in the detection element 23 as previously explained.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. An endovascular device (20) for inserting into a patient's blood vessel through a vascular access port comprising a near-field communication module (15) including an antenna (17), wherein at least the antenna of the near-field communication module is located within the vascular access port, the endovascular device comprising an electronic device (25) and an antenna arrangement (21) communicatively coupled to said electronic device for maintaining a near-field communication link between the electronic device and the near-field communication module whilst the endovascular device passes through the vascular access port.

2. The endovascular device (20) of claim 1, wherein the antenna arrangement (21) is located along a shaft of the endovascular device (20).

3. The endovascular device (20) of claim 2, wherein the antenna arrangement comprises an array of antenna elements (21), each of said antenna elements being communicatively coupled to said electronic device (25).

4. The endovascular device (20) of claim 3, wherein the endovascular device (20) further comprises a detection element (23) configurably coupled in between the electronic device (25) and the antenna elements (21), said detection element being adapted to detect the antenna element of said array proximal to the vascular access port (10) and to selectively connect said detected antenna element to the electronic device.

5. The endovascular device (20) of claim 4, wherein the detection element (23) is adapted to periodically or continually check which of said antenna elements (21) is proximal to the vascular access port (10).

6. The endovascular device (20) of claim 4 or 5, wherein the detection element (23) is adapted to detect the antenna element (21) of said array proximal to the vascular access port (10) based on the strength of an induced signal generated by said detected antenna element.

7. The endovascular device (20) of any of claims 1-6, wherein the electronic device (25) comprises a sensor or an actuator for interacting with the patient's tissue.

8. A vascular access port (10) for communicating with the endovascular device (20) of any of claims 1-7, the vascular access port comprising a near-field communication module (15) including an antenna (17), wherein at least the antenna of the near-field communication module is located within the vascular access port.

9. The vascular access port (10) of claim 8, wherein the near-field communication module (15) is arranged to generate a plurality of frequency bands in a frequency multiplex schema.

10. The vascular access port (10) of claim 8 or 9, further comprising a connection cable (18) for connecting the near-field communication module (15) to a user console (30) for the endovascular device (20).

11. An endovascular device arrangement (1) comprising the vascular access port (10) of any of claims 8-10 and the endovascular device (20) of any of claims 1-7 for inserting into a patient's blood vessel through said vascular access port.

12. An endovascular intervention system (100) including the endovascular device arrangement (1) of claim 11 and a user console (30) adapted to communicate with the electronic device (25) through the near-field communication module (15).

13. A method of operating the endovascular intervention system (100) of claim 12, the method comprising maintaining a near-field communication link between the near-field communication module (15) and the electronic device (25) during passing of the endovascular device (20) through the vascular access port (10).

14. The method of claim 13, wherein the near-field communication link is:
a unidirectional data communication from the electronic device (25) to the near-field communication module (15); and/or
an energy transfer from the near-field communication module to the electronic device.

15. The method of claim 13 or 14, further comprising identifying the antenna element (21) of the array of antenna elements proximal to the vascular access port (10), the method optionally further comprising selectively coupling said identified antenna element (21) to the electronic device (25).
